# EUROPEAN PATENT APPLICATION

(11) **EP 4 026 895 A1**
(43) Date of publication of application: **13.07.2022**
(21) Application number: 20882475.5
(22) Date of filing: 18.09.2020
(51) Int. Cl.: C12N 1/16, C12P 21/02, A23K 20/147, A23L 33/18, A61K 8/64, A61K 8/9728, A61K 36/064, A61K 38/06, C12R 1/865

(54) **YEAST STRAIN THAT PRODUCES GLUTATHIONE, AND GLUTATHIONE PRODUCTION METHOD USING SAME**

(30) Priority: 29.10.2019 KR 20190135659
(71) Applicant: CJ Cheiljedang Corporation, Seoul 04560 (KR)
(72) Inventor: HA, Cheol Woong, Seoul 04560 (KR); YANG, Eun Bin, Seoul 04560 (KR); KIM, Hyo Jin, Seoul 04560 (KR); KIM, Hyung Joon, Seoul 04560 (KR); IM, Yeong Eun, Seoul 04560 (KR)
(74) Representative: Regimbeau
(86) International application number: PCT/KR2020/012614
(87) International publication number: WO 2021/085854

(57) **Abstract**

Provided are a novel yeast strain producing glutathione and a method of producing glutathione using the same.

<110>     CJ CheilJedang Corporation

<120>     YEAST STRAIN PRODUCING GLUTATHIONE AND METHOD OF PRODUCING
          GLUTATHIONE USING THE SAME

<130>     OPA20065

<150>     KR 10-2019-0135659
<151>     2019-10-29

<160>     3

<170>     KoPatentIn 3.0

<210>     1
<211>     810
<212>     DNA
<213>     Unknown

<220>
<223>     CJ-5 18S(ITS, 5.8S) rRNA


<400>     1
gtgaactgcg gaggatcatt aagaatttaa taattttgaa aatggatttt ttttgttttg          60

gcaaaagcat gaaagctttt acggggcaaa aaaacacaag atggagagtc cacccgggcc         120

tgcgcttaaa tgcgcggtct tgctaggctt gtaagtttct ttcttgcgat tccaaacggt         180

gagagatttc tgtgcttttg ttataggaca attaaaaccg tttcaataca ccacactgtg         240

aagttttcat atctttgcaa ctttttcttt gggcattcaa gcaatcgggg cccaaaggta         300

acaaacacaa acaattttat ttattcatta aatttttgtc aaaaacaaaa attttcttaa         360

ctggaaattt taaaatatta aaaactttca acaacggatc tcttgtttct cccatcaata         420

aaaaacacac cgaaatgcga tacttaatgt gaattgcaga attccttgaa tcatcaaatc         480

tttgaacgca cattgcgccc cttggtattc ctgggggcag gccggtttga gcgtcatttc         540

cttctcaaac attctgtttg gtagtaaggg atactctttg gagttaactt gaaattgctg         600

gccttttctt tggaggtttt tttttccaaa aaaaggtttc tctgcgtgct tgaggtataa         660

tgcaagtacg gccgttttag gttttaccaa ctgcggctaa tcttttttat acagaccgta         720

ttgaaacgtt atctataaaa aaaaagcgtc taggcaaaca atgttcttaa agtttgacct         780

cacatcaggt aggagtaccc cctcaactta                                          810


<210>     2
<211>     806
<212>     DNA
<213>     Unknown

<220>
<223>     CJ-37 18S(ITS, 5.8S) rRNA


<400>     2

ctgcggagga tcttaagaat ttataatttt gaaaatggat ttttttgttt tggcaagagc 60

atgaaagctt ttactgggca agaagaccag aaatggagag tccagccggg cctgcgctta 120

agtgcgcggt cttgctaggc ctggaagttt ctttcttgct attccaaacg gtgaaagatt 180

tctgtgcttt tgttatagga caattaaaac cgtttcaata caacacactg tggagttttc 240

atatctttgc aactttttct ttgggcattc cagcaatcgg ggcccaaaag taacaaacac 300

aaacaatttt atttattcat taaatttttg tcaaaaacaa aaattttcct aactggaaat 360

tttaaaatat taaaaacttt caacaacgga tctcttggtt ctcgcatcga tgaagaacgc 420

aacgaaatgc catacgtaat gtgaattgca aaattccctg aatcatccaa tctttgaacg 480

cacattgcgc cccttggtat tccagggggc atgcctgttt gaacgtcatt tccttctcaa 540

acattctgtt tggtagtgag tgatactctt tggaattaac ttgaaattgc tggccttttc 600

attggatgtt ttttttccca aaaaaggttt ctctgcgtgc ttgaggtata atgcaagtac 660

gggcgtttta agttttacca actgcgggct aaactttttt atactgagcg tattggaacg 720

ttatccataa aaaaaaagcg tctaggcgaa caatgttctt aaagtttgac ctcaaatcac 780

gtaagaatac ccgctgaact taacca 806

<210> 3
<211> 814
<212> DNA
<213> Unknown

<220>
<223> S. cerevisiae YJM1592 18S(ITS, 5.8S) rRNA

<400> 3
gtgaacctgc ggaaggatca ttaaagaaat ttaataattt tgaaaatgga ttttttttgt 60

tttggcaaga gcatgagagc ttttactggg caagaagaca agagatggag agtccagccg 120

ggcctgcgct taagtgcgcg gtcttgctag gcttgtaagt ttctttcttg ctattccaaa 180

cggtgagaga tttctgtgct tttgttatag gacaattaaa accgtttcaa tacaacacac 240

tgtggagttt tcatatcttt gcaacttttt ctttgggcat tcgagcaatc ggggcccaga 300

ggtaacaaac acaaacaatt ttatttattc attaaatttt tgtcaaaaac aagaattttc 360

gtaactggaa attttaaaat attaaaaact ttcaacaacg gatctcttgg ttctcgcatc 420

gatgaagaac gcagcgaaat gcgatacgta atgtgaattg cagaattccg tgaatcatcg 480

aatctttgaa cgcacattgc gccccttggt attccagggg gcatgcctgt ttgagcgtca 540

tttccttctc aaacattctg tttggtagtg agtgatactc tttggagtta acttgaaatt 600

gctggccttt tcattggatg tttttttttc caaagagagg tttctctgcg tgcttgaggt 660

ataatgcaag tacggtcgtt ttaggtttta ccaactgcgg ctaatctttt ttatactgag 720

cgtattggaa cgttatcgat aagaagagag cgtctaggcg aacaatgttc ttaaagtttg 780

acctcaaatc aggtaggagt acccgctgaa ctta 814

## Description

### BACKGROUND OF THE INVENTION

### 1. Field of the Invention

The present disclosure relates a novel yeast strain producing glutathione and a method of producing glutathione using the same.

### 2. Description of the Related Art

Glutathione (GSH) is an organic sulfur compound that is most commonly present in cells, and it is in the form of a tripeptide in which three amino acids of glycine, glutamate, and cysteine are bound with one another.

In the body, glutathione exists in two forms: reduced glutathione (GSH) and oxidized glutathione (GSSG). Reduced glutathione (GSH), which exists in a relatively high percentage under general circumstances, is mainly distributed in the liver and skin cells of the human body and has important roles such as an antioxidant function of decomposing and removing oxygen radicals, a detoxification function of removing extrinsic compounds such as toxic substances, *etc.,* a whitening function of inhibiting melanin pigment production, *etc.*

Since production of glutathione gradually decreases with age, a reduction in the production of glutathione, which has important roles in antioxidant and detoxification functions, promotes accumulation of oxygen radicals, which is one of the main causes of aging, and therefore, an external supply of glutathione is needed (Sipes IG et al., The role of glutathione in the toxicity of xenobiotic compounds: metabolic activation of 1,2-dibromoethane by glutathione, Adv Exp Med Biol. 1986; 197: 457-67.).

Having such various functions, glutathione has attracted much attention as a material in various fields such as pharmaceuticals, health functional foods, cosmetics, *etc.,* and is also used in preparing flavoring ingredients, foods, and feed additives. It is known that glutathione has a significant effect on increasing the flavor of a raw material and maintaining a rich flavor, and glutathione can be used as a *kokumi* flavor enhancer by being used alone or in combination with other substances. Usually, *kokumi* substances have a richer flavor than existing *umami* substances such as nucleic acids, MSG, *etc.,* and are known to be produced by decomposition and aging of proteins.

However, despite the increasing demand for glutathione that may be used in various fields as described above, the market is not significantly activated because considerable costs are required for industrial production of glutathione.

There have been many efforts to solve the above problems, and as a result, the present inventors have developed a novel strain having an excellent glutathione-producing ability, thereby completing the present disclosure.

### SUMMARY OF THE INVENTION

An object of the present disclosure is to provide a novel *Saccharomyces cerevisiae* strain producing glutathione.

Another object of the present disclosure is to provide a method of preparing glutathione, the method including the step of culturing the strain.

Still another object of the present disclosure is to provide a method of preparing a composition including glutathione, the method including the steps of culturing the strain; and mixing an additive with one or more selected from the cultured strain, a dry product thereof, an extract thereof, a culture thereof, a lysate thereof, and glutathione collected therefrom.

Still another object of the present disclosure is to provide a composition for an antioxidant function, detoxification, immune enhancement, cosmetics, foods, or feeds, the composition including one or more selected from the strain; a dry product, extract, culture, and lysate of the strain; and glutathione collected from any one or more of the strain, dry product, extract, culture, and lysate.

Still another object of the present disclosure is to provide a composition for preparing a medical product which is used for preventing or treating a disease caused by glutathione deficiency or a pharmaceutical composition for preventing or treating a disease caused by glutathione deficiency, the composition including one or more selected from the strain; a dry product, extract, culture, and lysate of the strain; and glutathione collected from any one or more of the strain, dry product, extract, culture, and lysate.

### Advantageous Effects of the invention

A strain of the present disclosure can produce a significantly large amount of glutathione compared to existing glutathione-producing strains. Therefore, the strain of the present disclosure can be effectively used for the preparation of glutathione.

Further, a composition including one or more selected from the strain of the present disclosure; a dry product, extract, culture, and lysate of the strain; and glutathione collected from any one or more of the strain, dry product, extract, culture, and lysate has an excellent oxygen radical scavenging capacity, and thus the composition can be effectively used as a composition for an antioxidant function, detoxification, or immune enhancement.

Therefore, the composition can also be effectively used as a cosmetic composition, a medical composition, and a preparation thereof.

Further, when the composition is used in foods, it improves the above-described functions and seasoning property, and thus the composition may be usefully applied to preparation of a food composition and a feed composition.

### BRIEF DESCRIPTION OF THE DRAWINGS

FIG. 1 shows results of sensory evaluation of food compositions, each including an extract of *Saccharomyces cerevisiae* CEN.PK2-1D, CJ-5, or CJ-37 strain;
FIG. 2 shows results of confirming radical scavenging capacity of the extracts of *Saccharomyces cerevisiae* CEN.PK2-1D, CJ-5, and CJ-37 strains; and
FIG. 3 shows results of confirming oxygen radical absorbance capacity of *Saccharomyces cerevisiae* CEN.PK2-1D, CJ-5, and CJ-37 strains.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the present disclosure described herein. Further, these equivalents should be interpreted to fall within the present disclosure.

An aspect of the present disclosure may provide a novel *Saccharomyces cerevisiae* strain producing glutathione.

The strain producing glutathione of the present disclosure may be a *Saccharomyces cerevisiae* CJ-5 strain with Accession No. KCCM12568P.

The strain producing glutathione of the present disclosure may include an 18S (ITS, 5.8S) rRNA sequence having a homology or identity of 90% or higher, specifically, 91%, 92%, 93%, 93.2% or higher, 93.5% or higher, or 93.7% or higher to an 18S (ITS, 5.8S) rRNA sequence of a different *Saccharomyces cerevisiae* strain belonging to the same species of the same genus, specifically, a *Saccharomyces cerevisiae* strain of *Saccharomyces cerevisiae* YJM1592 (SEQ ID NO: 3), but the strain is not limited thereto. More specifically, the strain producing glutathione of the present disclosure may include an 18S (ITS, 5.8S) rRNA sequence having a homology or identity of 90% or more, specifically 91% or higher, 92% or higher, 93% or higher, 93.2% or higher, 93.5% or higher, or 93.7% or higher and an identity of less than 96%, less than 95.5%, or less than 95.2% to the 18S (ITS, 5.8S) rRNA sequence of YJM1592, but the strain is not limited thereto. The 18S (ITS, 5.8S) rRNA sequence is an internal transcribed spacer (ITS) sequence that exists between 18S and 5.8S rRNA, and is a sequence used for species-level classification.

The strain producing glutathione of the present disclosure may have an 18S (ITS, 5.8S) rRNA sequence of SEQ ID NO: 1. According to one exemplary embodiment, the 18S (ITS, 5.8S) rRNA sequence of the strain may have a homology or identity of 90% or higher, specifically, 95% or higher, 96% or higher, 97% or higher, 98% or higher, or 99% or higher to SEQ ID NO: 1, but the 18S (ITS, 5.8S) rRNA sequence of the strain is not limited thereto.

As used herein, the term "homology" or "identity" refers to a degree of matching between two given amino acid sequences or nucleotide sequences, and may be expressed as a percentage. The terms "homology" and "identity" may often be used interchangeably with each other.

The sequence homology or identity of a conserved polynucleotide or polypeptide may be determined by standard alignment algorithms, and default gap penalties established by a program being used may be used together. Actually, homologous or identical sequences may hybridize under moderately or highly stringent conditions such that the full length of the sequence or at least about 50%, 60%, 70%, 80%, or 90% of the full length of the sequence may hybridize. Additionally contemplated are polynucleotides that contain degenerate codons in place of codons in the hybridization.

Whether any two polynucleotide or polypeptide sequences have homology, similarity, or identity may be determined using known computer algorithms such as the "FASTA" program by using the default parameters as in Pearson et al., (1988) (Proc. Natl. Acad. Sci. USA 85: 2444), or determined using the Needleman-Wunsch algorithm (Needleman and Wunsch, 1970, J. Mol. Biol. 48: 443-453) as implemented in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J. et al., Nucleic Acids Research 12: 387 (1984)), BLASTP, BLASTN, FASTA (Atschul, S. F. et al., J. Mol. Biol. 215: 403 (1990); Guide to Huge Computers, Martin J. Bishop, ed., Academic Press, San Diego, 1994, and Carillo et al. (1988) SIAM J. Applied Math 48: 1073). For example, BLAST of the National Center for Biotechnology Information database or ClustalW may be used to determine homology, similarity, or identity.

Homology, similarity, or identity of polynucleotides or polypeptides may be determined, for example, by comparing sequence information using a GAP computer program such as Needleman et al. (1970), J. Mol. Biol. 48: 443, as disclosed in Smith and Waterman, Adv. Appl. Math (1981) 2: 482. Briefly, the GAP program defines similarity as the number of similar aligned symbols (*i.e*., nucleotides or amino acids), which are divided by the total number of symbols in the shorter of the two sequences. Default parameters for the GAP program may include: (1) a binary comparison matrix (containing a value of 1 for identities and 0 for non-identities) and the weighted comparison matrix of Gribskov et al. (1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10, a gap extension penalty of 0.5); and (3) no penalty for end gaps.

Further, whether any two polynucleotide or polypeptide sequences have homology, similarity, or identity may be determined by comparing sequences by Southern hybridization experiments under defined stringent conditions, and the defined appropriate hybridization conditions may be within the technical scope of the art and may be determined by a method well known to those skilled in the art.

The term "glutathione" of the present disclosure, which may be used interchangeably with "GSH", refers to a tripeptide consisting of three amino acids of glutamate, cysteine, and glycine. Glutathione may be used as a raw material of pharmaceuticals, health functional foods, flavoring ingredients, foods, feed additives, cosmetics, *etc.,* but is not limited thereto.

The novel *Saccharomyces cerevisiae* strain of the present disclosure is characterized by having a high glutathione-producing ability.

The strain may be a strain having an enhanced glutathione-producing ability, a strain having a high glutathione-producing ability, or a strain having an increased glutathione-producing ability.

The term "strain producing glutathione" of the present disclosure may be used interchangeably with the terms of "strain having a glutathione-producing ability", a "glutathione-producing strain", *etc.*

The *Saccharomyces cerevisiae* strain according to the present disclosure may include glutathione in an amount of 0.6% by weight or more, specifically, 0.7% by weight or more, 0.8% by weight or more, 0.9% by weight or more, 1.0% by weight or more, or 1.1% by weight or more, based on the dry weight of the strain, but the amount of glutathione included in the strain is not limited thereto.

Another aspect of the present disclosure may provide a composition for producing glutathione, wherein the composition includes the strain.

In the present disclosure, "glutathione production" or "glutathione preparation" may include accumulation of glutathione in the strain.

The composition for producing glutathione may be a composition capable of producing glutathione by the strain of the present disclosure, and for example, the composition may include the strain, and may further include an additional constitution capable of producing glutathione using the strain without limitation.

The composition for producing glutathione of the present disclosure may further include any appropriate additive commonly used in the composition for producing glutathione. The additive may be a naturally occurring or non-naturally occurring additive, but is not limited thereto.

The additive may include an excipient and/or an emulsifier. The excipient may be appropriately selected for use according to the intended use or form thereof, and may be, for example, one or more selected from starch, glucose, cellulose, lactose, glycogen, D-mannitol, sorbitol, lactitol, maltodextrin, calcium carbonate, synthetic aluminum silicate, calcium monohydrogen phosphate, calcium sulfate, sodium chloride, sodium hydrogen carbonate, purified lanolin, dextrin, sodium alginate, methylcellulose, colloidal silica gel, hydroxypropyl starch, hydroxypropylmethylcellulose, propylene glycol, casein, calcium lactate, primogel, gum arabic, and specifically, one or more selected from starch, glucose, cellulose, lactose, dextrin, glycogen, D-mannitol, and maltodextrin, but the excipient is not limited thereto. The emulsifier may be glycerol ester, sorbitan ester, monoglyceride, diglyceride, triglyceride, sucrose ester, sorbitan ester, propylene glycol ester, glycerin fatty acid ester, sorbitan fatty acid ester, propylene glycol fatty acid ester, sucrose fatty acid ester, lecithin, or a mixture thereof, but the emulsifier is not limited thereto, and those known in the art may be appropriately used.

The excipient may include, for example, a preservative, a wetting agent, a dispersing agent, a suspending agent, a buffering agent, a stabilizing agent, or an isotonic agent, but the excipient is not limited thereto.

Still another aspect of the present disclosure may provide a method of preparing glutathione, the method including a step of culturing the strain. Through culturing the strain, glutathione may be accumulated in the strain.

Media and other culture conditions used for culturing the strain of the present disclosure may be used without particular limitation as long as they are commonly used for culturing microorganisms of the genus *Saccharomyces.* Specifically, the strain of the present disclosure may be cultured in a common medium containing appropriate carbon sources, nitrogen sources, phosphorus sources, inorganic compounds, amino acids, and/or vitamins while controlling temperature, pH, *etc.* under aerobic or anaerobic conditions.

In the present disclosure, the carbon sources may include carbohydrates such as glucose, fructose, sucrose, maltose, *etc.;* sugar alcohols such as mannitol, sorbitol, *etc*.; organic acids such as pyruvate, lactate, citrate, *etc.;* and amino acids such as glutamic acid, methionine, lysine, *etc.,* but the carbon sources are not limited thereto. Additionally, natural organic nutrients such as starch hydrolysate, molasses, blackstrap molasses, rice bran, cassava, sugar cane molasses, and corn steep liquor may be used, and carbohydrates such as glucose and sterile pretreated molasses (*i.e*., molasses converted to a reducing sugar), *etc.* may be used. Furthermore, various other carbon sources may be used in a suitable amount without limitation. These carbon sources may be used alone or in a combination of two or more thereof, but are not limited thereto.

The nitrogen sources may include inorganic nitrogen sources such as ammonia, ammonium sulfate, ammonium chloride, ammonium acetate, ammonium phosphate, ammonium carbonate, ammonium nitrate, *etc.;* and organic nitrogen sources such as amino acids, peptone, NZ-amine, a meat extract, a yeast extract, a malt extract, corn steep liquor, casein hydrolysate, fish or decomposition products thereof, defatted soybean cake or decomposition products thereof, *etc.* These nitrogen sources may be used alone or in a combination of two or more thereof, but are not limited thereto.

The phosphorus sources may include potassium phosphate monobasic, dipotassium phosphate, corresponding sodium-containing salts, *etc.* The inorganic compounds may include sodium chloride, calcium chloride, iron chloride, magnesium sulfate, iron sulfate, manganese sulfate, calcium carbonate, *etc.*

Additionally, amino acids, vitamins, and/or suitable precursors may be included in the above medium. L-Amino acids, *etc.* may be added to the medium for culturing the strain. Specifically, glycine, glutamate, and/or cysteine may be added, and as needed, L-amino acids such as lysine may be further added, but are not necessarily limited thereto.

These media or precursors may be added to the culture in a batch or continuous mode, but are not limited thereto.

In the present disclosure, the pH of a culture may be adjusted during the culturing of a strain by adding a compound such as ammonium hydroxide, potassium hydroxide, ammonia, phosphoric acid, and sulfuric acid to the culture in an appropriate manner. Additionally, during the culturing, an anti-foaming agent such as a fatty acid polyglycol ester may be used to inhibit foam generation. Additionally, oxygen or an oxygen-containing gas may be injected into the culture in order to maintain an aerobic state of the culture; or nitrogen, hydrogen, or carbon dioxide gas may be injected, or no gas may be injected, in order to maintain an anaerobic or microaerobic state.

The temperature of a culture may be 25°C to 40°C, and more specifically, 28°C to 37°C, but the temperature is not limited thereto. The culturing may be continued until the desired amount of useful materials is obtained, specifically, for 1 to 100 hours, but the culturing time is not limited thereto.

The method of preparing glutathione may further include an additional process after the step of culturing. The additional process may be appropriately selected according to the use of glutathione.

Specifically, the method of preparing glutathione may further include a step of collecting glutathione from one or more materials selected from the strain, a dry product thereof, an extract thereof, a culture thereof, and a lysate thereof, after the step of culturing.

As used herein, the term "culture" may refer to a product resulting from culturing the strain of the present disclosure. For example, the culture may be a medium containing by-products which are generated by nutrient intake and metabolism during culturing the strain in the medium, and the culture may include all types of cultures which may be formed by using the medium, such as a diluted or concentrated liquid of the medium, a dry product obtained by drying the medium, a crude purified product or a purified product of the medium, or a mixture thereof, *etc.* The culture of the microorganism of the present disclosure may or may not include the microorganism. The culturing is the same as described above.

As used herein, the term "lysate" may refer to a product resulting from rupturing or lysing the strain or the culture thereof, or a supernatant obtained by centrifuging the lysate.

The culture, the lysate, the supernatant thereof, and fractions thereof are also included in the scope of the present disclosure. The method may further include a step of lysing the strain before or concurrently with the step of colleting. The lysing of the strain may be performed by a method commonly used in the technical field to which the present disclosure pertains, for example, by a buffer solution for lysis, a sonicator, heat treatment, a French press, *etc.* In addition, the step of lysing may include reactions of enzymes such as a cell wall degrading enzyme, a nuclease, a nucleotidyl transferase, a protease, *etc.,* but the step of lysing is not limited thereto.

With respect to the objects of the present disclosure, a dry yeast, yeast extract, yeast extract mix powder, or pure glutathione having a high content of glutathione may be prepared through the method of preparing glutathione, but the products that can be prepared therefrom are not limited thereto, and these products may be appropriately prepared according to the desired product.

As used herein, the term "dry yeast" may be used interchangeably with the term "dry product of the strain", *etc.* The dry yeast may be prepared by drying a yeast strain in which glutathione is accumulated, and specifically, may be included in a feed composition, a food composition, *etc*., but the method of preparing the dry yeast is not limited thereto.

As used herein, the term "yeast extract" may be used interchangeably with the term "strain extract", *etc.* The term strain extract may refer to a material which remains after the cell wall is separated from the cell body of the strain. Specifically, the strain extract may refer to remaining components, excluding the cell wall, obtained by lysis of the yeast cells. The strain extract may include glutathione and may include, as components other than glutathione, one or more components of proteins, carbohydrates, nucleic acids, and fibers, but the components to be included therein are not limited thereto.

The step of collecting may be performed using an appropriate method known in the art to thereby collect glutathione, which is the desired material.

The step of collecting may include a purification process. The purification process may be a process of separating only pure glutathione from the strain. Through the purification process, pure glutathione may be prepared.

As needed, the method of preparing glutathione may further include a step of mixing an additive with a material selected from the strain obtained after the step of culturing, the dry product, extract, culture, and lysate thereof, and glutathione collected therefrom. Through this step of mixing, a yeast extract mix powder may be prepared.

The additive may include an excipient and/or an emulsifier. The excipient and the emulsifier may be appropriately selected for use by those skilled in the art, and examples thereof are the same as described above.

Still another aspect of the present disclosure may provide a method of preparing a composition including glutathione, the method including the steps of culturing the strain; and mixing an additive with a material selected from the cultured strain, the dry product, extract, culture, and lysate thereof, and glutathione collected therefrom.

The composition of the present disclosure may further include a naturally occurring substance or a non-naturally occurring substance. The substance may include, but is not limited to, a pharmaceutically acceptable carrier, a sitologically acceptable carrier, a cosmetically acceptable carrier, *etc.,* depending on the intended use of the composition. The carrier may be appropriately selected by those skilled in the art based on known contents.

In the composition, the contents of the material selected from the strain of the present disclosure, the dry product, extract, culture, and lysate thereof, and glutathione collected therefrom may be 3% by weight to 30% by weight, based on the total weight of the composition.

The additive may include an excipient and/or an emulsifier. The excipient and the emulsifier may be appropriately selected for use by those skilled in the art, and examples thereof are the same as described above.

Still another aspect of the present disclosure may provide a composition for an antioxidant function, detoxification, immune enhancement, cosmetics, foods, or feeds, the composition including one or more selected from the strain; a dry product, extract, culture, and lysate of the strain; and glutathione collected from any one or more of the strain, dry product, extract, culture, and lysate.

Still another aspect of the present disclosure may provide a method of preparing the composition for an antioxidant function, detoxification, immune enhancement, cosmetics, foods, or feeds, the composition including one or more selected from the strain; a dry product, extract, culture, and lysate of the strain; and glutathione collected from any one or more of the strain, dry product, extract, culture, and lysate.

With respect to the objects of the present disclosure, the composition may include the strain itself, or may include a culture of the strain, a dry product of the strain, an extract of the strain, or a lysate of the strain, or may include glutathione collected from the culture, extract, dry product, or lysate of the strain, but is not limited thereto. In other words, since the strain, the dry product, extract, culture, or lysate thereof includes glutathione, glutathione may be included in any of these forms without limitation as long as the content of glutathione can be increased to a desired level, even though the type may differ depending on the specific use of the composition.

In the composition, a content of the material selected from the strain of the present disclosure, the dry product, extract, culture, and lysate thereof, and glutathione collected therefrom may be 3% by weight to 30% by weight, based on the total weight of the composition. In addition, the composition may further include any appropriate additive commonly used in compositions.

The composition may have an increased content of glutathione by including one or more materials selected from the strain producing glutathione of the present disclosure; the dry product, extract, culture, and lysate of the strain; and glutathione collected from any one of the strain, dry product, extract, culture, and lysate.

An aspect of the present disclosure may provide a composition for an antioxidant function, the composition including one or more materials selected from the strain; the dry product, extract, culture, and lysate of the strain; and glutathione collected from any one of the strain, dry product, extract, culture, and lysate.

As used herein, the term "antioxidant" may broadly refer to actions to inhibit, reduce, or control naturally occurring oxidation reactions, and more specifically, actions to inhibit, reduce, or control generation or reaction of free radicals generated in the body, hydrogen peroxide or peroxides generated from the free radicals, or hydroxyl radicals generated from the hydrogen peroxide.

An aspect of the present disclosure may provide a composition for detoxification, the composition including one or more materials selected from the strain; the dry product, extract, culture, and lysate of the strain; and glutathione collected from any one of the strain, dry product, extract, culture, and lysate.

As used herein, the term "detoxification" may refer to all actions to remove or reduce toxicity caused by harmful substances.

Glutathione, which is an endogenous antioxidant produced by cells, is not only directly involved in the neutralization of free radicals and oxygen radical compounds, but also maintains exogenous antioxidants such as vitamins C and E in a reduced form, and thus, the detoxification may include actions to inhibit, reduce, or control the generation or reaction of oxygen radicals and free radicals, hydrogen peroxide or peroxides generated from the free radicals, or hydroxyl radicals generated from the hydrogen peroxide. Further, since glutathione can protect thiol groups of cellular proteins, it may prevent side effects caused by overdose of drugs such as acetaminophen, *etc.,* and is also used for detoxification of methylglyoxal, which is produced as a metabolic by-product. Therefore, alleviation or reduction of toxicity of compounds may also be included in the scope of the detoxification.

An aspect of the present disclosure may provide a composition for immune enhancement, the composition including one or more materials selected from the strain; the dry product, extract, culture, and lysate of the strain; and glutathione collected from any one of the strain, dry product, extract, culture, and lysate.

As used herein, the "immune enhancement" refers to enhancing the body's defense against antigens, and specifically, may refer to increasing cellular and humoral immunity against antigens, and specifically, may refer to increased immunity, compared with immunity before administration of the composition. The mechanism of the immune enhancement is not limited, but may include, for example, those obtained by promoting the activity of antigen-presenting cells such as macrophages, *etc.,* or promoting a specific activity for lymphocytes.

As described above, glutathione has a strong antioxidant capacity, has the roles of improving liver functions and helping decomposition of toxic substances in the body, and removes harmful oxygen radicals and participates in the immune system. That is, glutathione increases activities of T lymphocytes and leukocytes to improve the body's immunity and to rejuvenate individuals debilitated due to various chronic diseases, *etc.* Therefore, one or more materials selected from the strain including glutathione of the present disclosure; the dry product, extract, culture, and lysate of the strain; and glutathione collected from any one of the strain, dry product, extract, culture, and lysate can be effectively used for immune enhancement.

An aspect of the present disclosure may provide a composition for cosmetics, the composition including one or more materials selected from the strain; the dry product, extract, culture, and lysate of the strain; and glutathione collected from any one of the strain, dry product, extract, culture, and lysate.

The "composition for cosmetics" of the present disclosure may be prepared into a formulation selected from the group consisting of a solution, an ointment for external use, a cream, a foam, a nutrition emollient, a soft emollient, a pack, an emollient water, a milky lotion, a makeup base, an essence, a liquid cleansing agent, a bath preparation, a sunscreen cream, a sun oil, a suspension, an emulsion, a paste, a gel, a lotion, a powder, a soap, a surfactant-containing cleansing agent, an oil, a powder foundation, an emulsion foundation, a wax foundation, a patch, and a spray, but is not limited thereto.

The composition for cosmetics may further include one or more cosmetically acceptable carriers that are blended in general skin cosmetics, and may be appropriately blended with, as usual ingredients, for example, oil, water, a surfactant, a moisturizer, lower alcohol, a thickener, a chelating agent, a pigment, a preservative, a perfume, *etc.,* but the cosmetically acceptable carriers are not limited thereto. The cosmetically acceptable carrier included in the composition for cosmetics of the present disclosure may be appropriately selected by those skilled in the art according to the formulation of the cosmetic composition.

An aspect of the present disclosure may provide a composition for foods, the composition including one or more materials selected from the strain; the dry product, extract, culture, and lysate of the strain; and glutathione collected from any one of the strain, dry product, extract, culture, and lysate.

In one specific embodiment, the composition for foods may include the cell body or the extract of the strain of the present disclosure, and more specifically, may be prepared for use in an appropriate formulation by mixing the strain extract with an additive, according to the food to be applied, but is not limited thereto. The strain extract is the same as described above.

The additive may include an excipient and/or an emulsifier. The excipient and the emulsifier may be appropriately selected for use by those skilled in the art, and examples thereof are the same as described above. In addition, an auxiliary food additive, a food stabilizer, a water retention agent, *etc.* may be included.

The "composition for foods" of the present disclosure may include all types of functional foods, nutritional supplements, health foods, and food additives.

The above types of the composition for foods may be prepared in various forms according to a common method known in the art.

The composition for foods may be prepared in the forms of pills, powders, granules, infusions, tablets, capsules, liquids, *etc.* The foods, to which the composition of the present disclosure may be added, may include, for example, various foods, such as rice, edible grain powders (edible grain flours), grain soups, bowl of rice served with toppings, noodles, rice soups, instant rice, condiments, rice in a lunch box, dry cooked rice, bread, edible sugars, rice cakes, mixing sauces, sauces, spices, edible salts, seasonings, seasoning powders, processed, frozen, dried, and cooked fruits and vegetables, jellies, jams, candied fruits, eggs, milk and other dairy products, edible fats and oils, coffee, cocoa and coffee substitutes, tapioca, grain flours and grain preparations, ramens, udon, noodles, chopped noodles, cold noodles, porridge, soups, soup dishes, instant foods, frozen foods, retort foods, other beverages, gums, teas, vitamin complexes, health supplements, *etc*., but the forms of food preparation are not limited thereto.

As an ingredient that may be included in the composition for foods of the present disclosure, additional ingredients such as various herbal extracts, auxiliary food additives, or natural carbohydrates may be included as in ordinary foods. In addition, the auxiliary food additive may include auxiliary food additives common in the art, for example, flavoring agents, flavor enhancers, coloring agents, fillers, stabilizers, *etc.*

Examples of the natural carbohydrate include monosaccharides, for example, glucose, fructose, *etc*.; disaccharides, for example, maltose, sucrose, *etc.;* and polysaccharides, for example, common sugars such as dextrin, cyclodextrin, *etc.,* and sugar alcohols such as xylitol, sorbitol, erythritol, *etc.* In addition to those described above, natural flavoring agents (for example, rebaudioside A, glycyrrhizin, *etc*.) and synthetic flavoring agents (saccharin, aspartame, *etc*.) may be advantageously used as flavoring agents. In addition, common food additives used for the purpose of supplementing taste and nutrition, for example, nucleic acids, amino acids, organic acids, *etc.* may be added.

In addition to those described above, the composition for foods of the present disclosure may include many nutritional supplements, vitamins, minerals (electrolytes), flavoring agents such as synthetic flavoring agents and natural flavoring agents, coloring agents, and fillers (cheese, chocolate, *etc*.), pectic acid and salts thereof, alginic acid and salts thereof, organic acids, protective colloidal thickening agents, pH regulating agents, stabilizing agents, preservatives, glycerin, alcohol, carbonating agents used in carbonated beverages, *etc.* In addition, the composition may include fruit pulp for the preparation of natural fruit juices, fruit juice drinks, and vegetable drinks. These ingredients may be used alone or in combination.

The foods of the present disclosure may be prepared by a method commonly used in the art, and during the preparation, raw materials and ingredients commonly added in the art may be added for the preparation. In addition, the foods may be into any formulation without limitation as long as the formulation is recognized as foods.

Further, when the food composition of the present disclosure is used as a health functional food, the food composition of the present disclosure may be prepared in various forms of formulations. The food composition of the present disclosure has advantages in that it has no side effects that may occur when drugs are administered for a long period of time, because the food composition of the present disclosure is prepared using a raw material, unlike general drug, and that it has excellent portability. Therefore, the food of the present disclosure may be consumed as a supplement.

Meanwhile, the composition for foods of the present disclosure may also include flavoring agents, flavor enhancers, coloring agents, fillers, stabilizers, and flavors that may also be classified as food additives.

As used herein, the term "flavor" may be a material added to enhance the flavor of foods. The flavor may be a material that allows foods to have an excellent seasoning property.

The flavor may be classified according to taste components. In other words, the flavor may be classified into a neutral flavor, a beef flavor, a chicken flavor, a pork flavor, a *kokumi* flavor, *etc.,* depending on the flavor.

The term "*kokumi* flavor" refers to a flavor which releases the flavor of *kokumi.* The term "*kokumi*" is a Japanese word, and can also be expressed as 'mouthfulness', 'continuity', 'thickness', and 'heartiness' in English, and expressed as 'rich taste', 'thick taste', 'mouth-filling taste', 'dense taste', 'sticky taste', *etc.* in Korean. The 'neutral flavor' refers to a flavor that maximizes '*umami*' and minimizes other flavors to produce a mild and clean flavor. For example, oils such as canola oil or grapeseed oil among oils may be acknowledged as having a neutral flavor. The term 'seasoning property' may mean to have a sour, sweet, salty, bitter, or *umami* taste, but is not limited thereto.

The composition for foods of the present disclosure may include 3% by weight to 30% by weight of the yeast extract of the present disclosure, based on the total weight of the composition, and may further include one or more selected from ammonium chloride, maltodextrin, crystalline powdered glucose, and an emulsifier. In a specific embodiment, the food composition may include 3% by weight to 25% by weight of the yeast extract of the present disclosure, 20% by weight to 35% by weight of ammonium chloride, 25% by weight to 35% by weight of maltodextrin, 3% by weight to 7% by weight of crystalline powdered glucose, and 0.1% by weight to 1% by weight of an emulsifier, based on the total weight of the composition, but the food composition is not limited thereto.

An aspect of the present disclosure may provide a composition for feeds, the composition including one or more materials selected from the strain; the dry product, extract, culture, and lysate of the strain; and glutathione collected from any one of the strain, dry product, extract, culture, and lysate.

Specifically, the composition for feeds may include any one or more of the strain of the present disclosure as it is, the dry product of the strain, and the extract of the strain, or may include cell wall components obtained during a process of preparing the extract of the strain, and for example, the dry product of the strain (dry yeast) and/or the extract of the strain (yeast extract), but is not limited thereto.

The "composition for feeds" of the present disclosure is any appropriate natural or artificial diet, single meal, *etc.,* for animals to eat, ingest, and digest, or components of the single meal. The feed including the yeast having an increased content of glutathione according to the present disclosure may be prepared in various forms of feeds known in the art, and specifically, concentrated feeds, crude feeds, and/or special feeds may be included.

The type of the feed is not particularly limited, but any feed that is commonly used in the art may be used. Non-limiting examples of the feed may include plant-based feeds such as grains, root plants, food processing by-products, seaweed, fibers, drug by-products, oils, starches, meals, grain by-products, *etc*.; and animal-based feeds such as proteins, inorganics, minerals, fats and oils, single-cell proteins, zooplankton, foods, *etc.* These feeds may be used alone or in combination of two or more thereof.

Another aspect of the present disclosure may provide a composition for preparing a medical product which is used for preventing or treating a disease caused by glutathione deficiency, the composition including one or more selected from the strain; a dry product, extract, culture, and lysate of the strain; and glutathione collected from any one or more of the strain, dry product, extract, culture, and lysate.

Still another aspect of the present disclosure may provide a pharmaceutical composition for preventing or treating a disease caused by glutathione deficiency, the composition including one or more selected from the strain; a dry product, extract, culture, and lysate of the strain; and glutathione collected from any one or more of the strain, dry product, extract, culture, and lysate.

As used herein, the term "pharmaceutical composition" may refer to a chemical/biological compound or substance, or a mixture or combination of two or more such compounds or substances, intended for use in the medical diagnosis, cure, treatment, or prevention of a disease or pathology.

As used herein, the term "composition for preparing a medical product" may be used in the same meaning as the pharmaceutical composition, or may refer to a composition, which further includes any additional ingredients needed for the preparation and/or formulation of a medical product. However, the composition is not limited thereto.

As described above, glutathione contributes to the antioxidant functions of decomposing and removing oxygen radicals, detoxification function, and immune enhancement. Thus, the strain, or the culture, dry product, extract, or lysate thereof including glutathione at high concentrations may be effectively used for the preparation of a medical product, which may be effectively used for treating a disease caused by glutathione deficiency.

Further, since the strain, or the culture, dry product, extract, or lysate thereof includes glutathione at high concentrations, these may be included, as they are, in a pharmaceutical composition for preventing or treating a disease caused by glutathione deficiency.

The disease is not limited, as long as it is a disease caused by glutathione deficiency. For example, the disease includes any disease without limitation, as long as it is caused by accumulation of oxygen radicals and/or immunity decline, and specifically, atherosclerosis; neurodegenerative diseases including Lou Gehrig's disease, Parkinson's disease, Alzheimer's disease, amyotrophic lateral sclerosis, and Huntington's disease; cardiovascular diseases including myocardial infarction, angina, coronary artery disease, and ischemic heart disease; ischemic brain diseases including stroke; digestive disorders including diabetes, gastritis, and gastric cancer; cancers; leukemia; cataract; aging; rheumatoid arthritis; hepatitis; atopic dermatitis; *etc.,* but the disease is not limited thereto.

The medical and/or pharmaceutical composition may further include a pharmaceutically acceptable carrier. As used herein, the term "pharmaceutically acceptable" may mean properties of being non-toxic to cells or humans exposed to the medical product. Any carrier may be used without limitation as long as it is known in the art, such as a buffering agent, a preservative, an analgesic agent, a solubilizing agent, an isotonic agent, a stabilizer, a base agent, an excipient, a lubricant, *etc.*

In addition, the medical and/or pharmaceutical composition may be formulated in oral dosage forms such as powders, granules, tablets, capsules, suspensions, emulsions, syrups, and aerosols; external preparations; suppositories; and sterile injectable solutions according to common methods. Furthermore, they may be used in the form of an external skin preparation, such as an ointment preparation, a lotion preparation, a spray preparation, a patch preparation, a cream preparation, a powder preparation, a suspension preparation, a gel preparation, or a gel. Carriers, excipients, and diluents that may be included in the medical product of the present disclosure include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. When formulated, they are prepared using diluents or excipients such as fillers, extenders, binders, wetting agents, disintegrants, surfactants, *etc.,* which are commonly used.

Solid preparations for oral administration include a tablet, a pill, powders, granules, and a capsule, and such solid preparations may be prepared by mixing the composition with at least one excipient, for example, starch, calcium carbonate, sucrose, lactose, gelatin, *etc.* In addition to the simple excipients, lubricants such as magnesium stearate and talc are also used. Liquid preparations for oral administration include a suspension, a liquid for internal use, an emulsion, a syrup, *etc.,* and various excipients such as a wetting agent, a sweetener, a flavor, a preservative, *etc.* may be included, in addition to simple diluents commonly used, such as water and liquid paraffin. Preparations for parenteral administration include a sterile aqueous solution, a non-aqueous solvent, a suspension, an emulsion, a lyophilized agent, and a suppository. As the non-aqueous solvent and suspension, propylene glycol, polyethylene glycol, plant oil such as olive oil, injectable ester such as ethyloleate, *etc.* may be used. As a suppository base, witepsol, macrogol, tween 61, cacao butter, lauric butter, glycerogelatin, *etc.* may be used.

The medical and/or pharmaceutical composition may be administered in a pharmaceutically effective amount. As used herein, the term "administration" refers to introduction of a predetermined substance to an individual by an appropriate method. The composition may be administered through any general route as long as it is able to reach the target tissue, and the administration route may include intraperitoneal administration, intravenous administration, intramuscular administration, subcutaneous administration, intradermal administration, oral administration, topical administration, intranasal administration, intrapulmonary administration, and rectal administration, but the administration route is not limited thereto.

The term "individual" may refers to all animals including humans, rats, mice, livestock, *etc.* Specifically, it may be a mammal including a human.

The term "pharmaceutically effective amount" refers to an amount sufficient to treat a disease at a reasonable benefit/risk ratio applicable to any medical treatment without side effects. An effective dose level may be easily determined by those skilled in the art, depending on factors including a patient's sex, age, body weight, health conditions, the type of disease, severity, drug activity, sensitivity to drugs, administration method, administration time, administration route, discharge rate, treatment period, and drugs used in combination or simultaneously, and other factors well known in the medical field. The above recommended dose may be administered once daily or in several divided doses.

Still another aspect of the present disclosure may provide use of the strain in producing glutathione.

The strain is the same as described above.

### EXAMPLES

Hereinafter, the present disclosure will be described in more detail with reference to Examples and Experimental Examples. However, these Examples and Experimental Examples are for illustrative purposes only, and the scope of the present disclosure is not intended to be limited by these Examples and Experimental Examples.

### Example 1: Selection of strain having excellent glutathione production and verification of glutathione-producing ability

Strains were obtained from a Korean traditional *Nuruk* containing various strains, and the strains were improved to select a strain having high glutathione-producing ability.

In detail, grain samples such as rice, barley, mung beans, oats, *etc.* were collected from a total of 20 areas including Yongin, Icheon, Pyeongtaek, Hwaseong, *etc.* in Gyeonggi Province, Korea, and a Korean traditional *Nuruk* was prepared. The collected grain samples were pulverized, kneaded, wrapped in a cloth, pressed firmly to form a shape, wrapped with straw, and fermented for 10 days, and then slowly dried to prepare a Korean traditional *Nuruk.* To isolate various strains from the prepared Korean traditional *Nuruk*, experiments were performed as follows. 45 mL of saline was added to 5 g of the Korean traditional *Nuruk*, and pulverized with a mixer. For pure isolation of yeast strains, serial dilution was performed, and the resultant was spread on YPD Agar (10 g/L of yeast extract, 20 g/L of bacto peptone, 20 g/L of glucose, based on 1 liter of distilled water), and cultured at 30°C for 48 hours. Through examination of colony morphology and microscopic verification, yeast colonies were each streaked on YPD agar. 25 mL of YPD broth was dispensed into a 250 mL Erlenmeyer flask, and pure isolated strains were inoculated and cultured with shaking (30°C, 200 rpm) for 48 hours to examine the production amount of glutathione, followed by screening of the strains.

In order to improve the primary isolated strains, a random mutation was induced in each isolated strain. Among the yeast strains isolated from the Korean traditional *Nuruk*, a strain having 15 mg/L of glutathione production was selected and named as CJ-37 strain. The CJ-37 strain was cultured on a solid medium and then inoculated into a broth to obtain a culture. The yeast cells were irradiated with UV using a UV lamp. Thereafter, the UV-irradiated culture broth was plated on a plate medium and only the mutant strains that formed colonies were isolated, and the amount of glutathione production of these mutant strains was examined.

As a result, it was confirmed that the glutathione production of these mutant strains was up to 105 mg/L, and the strain showing the highest amount of glutathione production (105 mg/L) was selected as the glutathione-producing strain and named as CJ-5 strain, which was deposited under the Budapest Treaty in the international depository authority Korean Culture Center of Microorganisms (KCCM) on July 31, 2019, with Accession No. KCCM12568P.

### Example 2: Comparison of glutathione-producing ability

In order to confirm the glutathione-producing ability of the CJ-5 strain selected in Example 1, three kinds of known microorganisms (Table 1) were cultured by the following method and then the GSH-producing ability was compared and analyzed. As a control, the CJ-37 strain obtained in Example 1 was also cultured and analyzed for comparison.

**[Table 1]**

| Strain |
|---|
| *1. Candida utilis* KCCM50667 |
| *2. Kluyveromyces lactis* ATCC8585 |
| *3. Saccharomyces cerevisiae* CEN.PK2-1D |

First, each strain was cultured at 30°C for 1 day, and then inoculated into a 250 mL corner-baffle flask containing 25 mL of a YPD medium (10 g/L of yeast extract, 20 g/L of bacto peptone, 20 g/L of glucose, based on 1 liter of distilled water). Amino acids (L-cysteine, L-glutamic acid, and L-glycine) were added at a concentration of 20 mM at the time point of 16 hours from the start point of the culture, and cultured at 30°C at 200 rpm.

To measure GSH concentrations, 500 µL of the culture sample containing cells was lysed at 80°C at 800 rpm for 10 minutes, and then GSH concentrations were measured using an Abcam GSH Ration Detection Assay. The dry weight was calculated by converting OD values, and used to calculate GSH content (%) = GSH weight/yeast dry weight. The results are shown in Table 2.

**[Table 2]**

| Strain evaluation | | | | |
|---|---|---|---|---|
| Strain | 16 Hr | 24 Hr | | |
| | OD₆₀₀ | OD₆₀₀ | GSH (mg/L) | GSH content (%) |
| *Candida utilis* KCCM50667 | 72.0 | 65.8 | 80.3 | 0.59 |
| *Kluyveromyces lactis* ATCC8585 | 81.0 | 74.2 | 99.0 | 0.65 |
| *Saccharomyces cerevisiae* CEN.PK2-1D | 28.9 | 39.0 | 31.3 | 0.39 |
| CJ-37 | 35.4 | 37.2 | 14.6 | 0.19 |
| CJ-5 | 41.0 | 46.0 | 105.2 | 1.11 |

As a result of evaluating the GSH-producing ability of 5 types of yeasts, it was confirmed that the CJ-5 strain had the highest GSH content, based on the weight of the cells. Specifically, it was confirmed that the CJ-5 strain had the highest GSH content of about 200% to about 300%, based on the weight of the yeast cells, compared to existing GSH-producing strains (*Candida utilis* KCCM50667 strain, *Kluyveromyces lactis* ATCC8585 strain, and *Saccharomyces cerevisiae* CEN.PK2-1D), and in particular, the GSH producing ability of CJ-5 strain was improved by about 584%, compared to CJ-37.

These results confirmed that the CJ-5 strain has an excellent GSH-producing ability, suggesting that the strains of the present disclosure may be effectively used for GSH production.

### Example 3: Verification of 18S (ITS, 5.8S) rRNA of CJ-5 strain by gene sequencing

To confirm the classification of the CJ-5 strain having the highest glutathione production and the CJ-37 strain having the lowest glutathione production, which were isolated in Example 1, 18S (ITS, 5.8S) rRNA sequences of these two strains were compared and analyzed.

In detail, the similarity in the 18S (ITS, 5.8S) ribosomal RNA (rRNA) sequence between the two strains was compared using the BLAST database (http://www.ncbi.nlm.nih.gob/blast/). The 18S(ITS, 5.8S) rRNA sequence of CJ-5 strain is represented by SEQ ID NO: 1, the 18S (ITS, 5.8S) rRNA sequence of CJ-37 strain is represented by SEQ ID NO: 2, and the 18S (ITS, 5.8S) rRNA sequence of *Saccharomyces cerevisiae* YJM1592 chromosome XII is represented by SEQ ID NO: 3.

As a result, the CJ-5 strain showed a similarity of about 93.73% to the 18S (ITS, 5.8S) rRNA sequence of *Saccharomyces cerevisiae* YJM1592 chromosome XII. Meanwhile, the CJ-37 strain also showed a similarity of about 95.20% to the 18S (ITS, 5.8S) rRNA sequence of *Saccharomyces cerevisiae* YJY1592 chromosome XII.

These results indicate that both the CJ-5 strain and the CJ-37 strain are *Saccharomyces cerevisiae* strains. Combining these results with the experimental results of Example 2, it can be interpreted that the CJ-5 strain of the present disclosure has a GSH-producing ability about 6-fold higher than those of the microorganism belonging to the same species of the same genus as well as to the microorganism belonging to a different species of a different genus, and also has a GSH-producing ability of 284% (*i*.*e*., about 3-fold) higher compared to that of *Saccharomyces cerevisiae* CEN.PK2-1D, which is another microorganism belonging to the same species of the same genus.

These results suggest that the strain of the present disclosure has a high GSH-producing ability, and thus the strain, the dry product, extract, culture and/or lysate thereof may be used in various cosmetics, foods, and feeds which are prepared using GSH as a raw material.

### Example 4: Preparation of composition including glutathione

In order to prepare a yeast extract including glutathione, *Saccharomyces cerevisiae* CEN.PK2-1D, and CJ-5 and CJ-37 yeast cultures were first centrifuged using a centrifuge.

Each of the centrifuged yeasts was washed twice and subjected to repeated centrifugation to obtain each yeast. Thereafter, a cell wall degrading enzyme, a nuclease, a nucleotidyl transferase, and a protease were added, and an enzymatic reaction was performed at pH 5.2 and a temperature of 45°C for 48 hours.

After the enzymatic reaction, the enzymes in the supernatant were inactivated by heat-treatment at 85°C for 30 minutes, and each enzyme-inactivated supernatant was concentrated to prepare a yeast extract.

As a result, *Saccharomyces cerevisiae* CEN.PK2-1D, CJ-5 and CJ-37 yeast extracts were shown to include glutathione of about 0.8%, 2.2%, and 0.4%, based on the total weight.

### Example 5: Sensory evaluation of food composition including glutathione

The food compositions including the yeast extracts prepared in Example 4 were subjected to sensory evaluation. In detail, for sensory evaluation of *Saccharomyces cerevisiae* CEN.PK2-1D, CJ-5 and CJ-37 yeast extracts, sensory evaluation was performed using a *consommé* chicken soup as a base.

Sensory evaluation was performed for sensory testers by using a *consommé* chicken soup mixture containing 5% each of the yeast extracts. The evaluation was performed with respect to taste persistence (*kokumi*), savory taste (*umami*), bitter taste (off-flavor), increase in chicken taste, soft and deep taste, and overall preference.

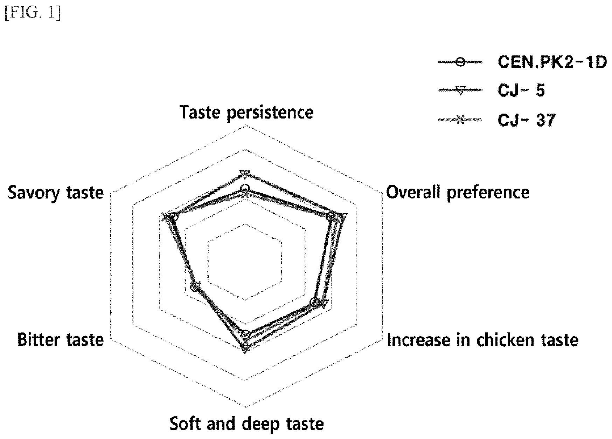

As a result, it was confirmed that the CJ-5 yeast extract had superior taste persistence (*kokumi*) and a softer and deeper taste compared to the CEN.PK2-1D yeast extract and the CJ-37 yeast extract, and the overall preference of the CJ-5 yeast extract was increased (FIG. 1).

These results confirmed that the yeast extract of the present disclosure may be effectively used for the preparation of a food composition having an excellent seasoning property, and thus one or more selected from the yeast strain; the dry product, extract, culture, and lysate of the strain; and glutathione collected from any one or more of the strain, dry product, extract, culture, and lysate may also be effectively used for the preparation of food compositions.

### Example 6: Verification of antioxidant effect of composition including glutathione

### Example 6-1: Test of radical scavenging capacity

The radical scavenging capacity of the yeast extracts including glutathione was evaluated.

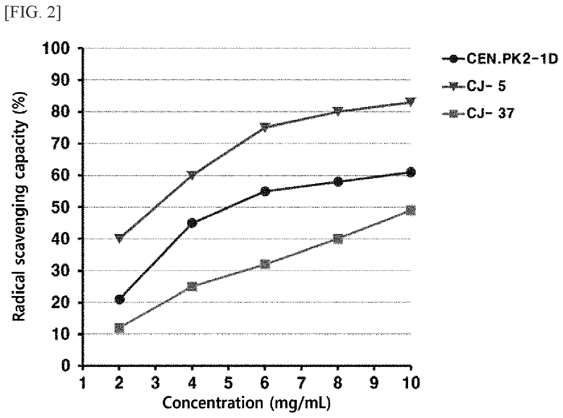

In detail, 5 mL of 0.1 mM 1,1-diphenyl-2-picrylhydrazyl was added to each 5 mL of the yeast extract suspensions of *Saccharomyces cerevisiae* CEN.PK2-1D, CJ-5, and CJ-37 prepared in Example 4, and allowed to react for 30 minutes in a dark place. Then, the absorbance was measured at 517 nm. In the concentration range of 2 mg/mL to 10 mg/mL, the CEN.PK2-1D, CJ-5, and CJ-37 extracts showed a radical scavenging capacity of 21% to 61%, 40% to 83%, and 12% to 49%, respectively (FIG. 2). At a concentration of 10 mg/mL, the antioxidant activity of the CJ-5 yeast extract having a high content of glutathione was increased up to 1.7 times, compared to those of CEN.PK2-1D and CJ-37 yeast extracts.

### Example 6-2: Test of oxygen radical absorbance capacity

To evaluate the antioxidant activity of the yeast extracts including glutathione, oxygen radical absorbance capacity (ORAC) was analyzed.

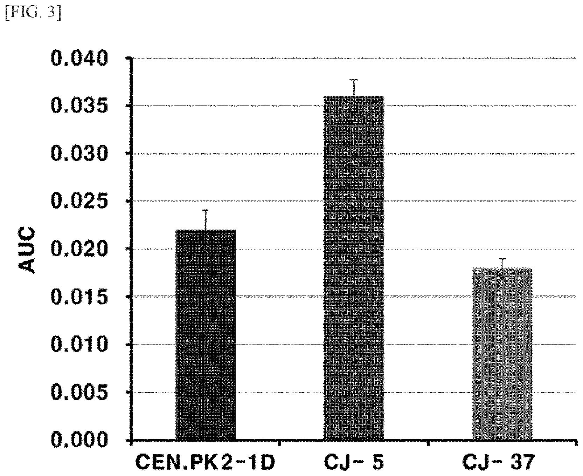

In detail, each 5 mL of the yeast extract suspensions of *Saccharomyces cerevisiae* CEN.PK2-1D, CJ-5 and CJ-37 prepared in Example 4 was mixed with 40 mL of fluorescein, and then allowed to react at 37°C for 15 minutes. 25 mL of 2,2'-azobis-(2-amidinopropane) HCl was added thereto, and the absorbance was measured at 485 nm and 528 nm for 60 minutes. The oxygen radical absorbance capacity was compared by obtaining the area under the curve (AUC). The oxygen radical absorbance capacity of the CJ-5 yeast extract was higher compared to those of the CEN.PK2-1D yeast extract and the CJ-37 yeast extract (FIG. 3). As described above, the CJ-5 yeast extract of the present disclosure showed a significantly high antioxidant activity compared to the CEN.PK2-1D and CJ-37 yeast extracts.

These results confirmed that the yeast extract exhibited excellent antioxidant activity, and thus one or more selected from the yeast strain; the dry product, extract, culture, and lysate of the strain; and glutathione collected from any one or more of the strain, dry product, extract, culture, and lysate may also be effectively used for anti-oxidation, detoxification, and immune enhancement.

### Preparation Example 1: Preparation of food composition including yeast extract

From the Experimental Examples above, the unique *umami* taste and an effect of controlling off-flavors and off-odors of yeast extract were confirmed, and thus a food composition including the yeast extract of the present disclosure was prepared.

In detail, a yeast extract mixed powder having a content ratio of 3% to 30% of the yeast extract, 20% to 40% of ammonium chloride, 20% to 40% of maltodextrin, 1% to 10% of crystalline powdered glucose, and 0.1% to 1% of an emulsifier was prepared.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

[stamp]

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Saccharomyces cerevisiae CJ-5 | Accession number given by the
INTERNATIONAL DEPOSITARY AUTHORITY:
KCCM12568P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on July. 31. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: July. 31. 2019.

[stamp: 한국미생물보존센터장인]

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A *Saccharomyces cerevisiae* strain producing glutathione with Accession No. KCCM12568P.

2. The strain of claim 1, wherein the strain includes a nucleotide sequence of SEQ ID NO: 1 as an 18S rRNA sequence.

3. A method of producing glutathione, the method comprising the step of culturing the strain of claim 1.

4. The method of claim 3, further comprising the step of collecting glutathione from one or more materials selected from the cultured strain, a dry product of the strain, an extract of the strain, a culture of the strain, and a lysate of the strain.

5. A method of preparing a composition including glutathione, the method comprising the steps of:
culturing the strain of claim 1; and
mixing an additive with one or more materials selected from the cultured strain, a dry product of the strain, an extract of the strain, a culture of the strain, a lysate of the strain, and glutathione collected therefrom.

6. The method of claim 5, wherein the additive includes an excipient or an emulsifier.

7. The method of claim 5, wherein the amount of one or more materials selected from the cultured strain, a dry product of the strain, an extract of the strain, a culture of the strain, a lysate of the strain, and glutathione collected therefrom is 3% by weight to 30% by weight, based on the total weight of the composition.

8. A composition for an antioxidant function, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

9. A composition for detoxification, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

10. A composition for immune enhancement, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

11. A composition for cosmetics, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

12. A composition for foods, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

13. A composition for feeds, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

14. A composition for preparing a medical product which is used for preventing or treating a disease caused by glutathione deficiency, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.

15. A pharmaceutical composition for preventing or treating a disease caused by glutathione deficiency, the composition comprising one or more materials selected from the strain of claim 1; a dry product of the strain, an extract of the strain, a culture of the strain, and a lysate of the strain; and glutathione collected from any one or more of the strain, the dry product, the extract, the culture, and the lysate.
